# EUROPEAN PATENT SPECIFICATION

(11) **EP 0 820 462 B1**
(45) Date of publication and mention of the grant of the patent: **03.03.1999**
(21) Application number: 96916913.5
(22) Date of filing: 31.05.1996
(51) Int. Cl.: C07H 21/00, C12N 15/11, A61K 31/70

(54) **HYDROLYSIS AND PHOTOCLEAVAGE OF NUCLEIC ACID USING A METAL COMPLEX**
HYDROLYSE UND PHOTOSPALTUNG VON NUKLEINSÄURE UNTER VERWENDUNG VON METALLKOMPLEXEN
HYDROLYSE ET PHOTOCLIVAGE D'ACIDE NUCLEIQUE AU MOYEN DE COMPLEXES METALLIQUES

(30) Priority: 02.06.1995 US 458347
(43) Date of publication of application: 28.01.1998
(73) Proprietor: PHARMACYCLICS, INC., Sunnyvale, CA 95086-4521 (US); BOARD OF REGENTS THE UNIVERSITY OF TEXAS SYSTEM, Austin, Texas 78701 (US)
(72) Inventor: MAGDA, Darren, Cupertino, CA 95014 (US); SESSLER, Jonathan, L., Austin, TX 78731 (US)
(74) Representative: Raynor, John
(86) International application number: PCT/US96/08262
(87) International publication number: WO 96/38461

(56) References cited:
- EP-A- 0 214 908
- WO-A-90/02747
- WO-A-94/29316
- WO-A-95/21845
- WO-A-96/09315
- FR-A- 2 697 254
- BIOCONJUGATE CHEMISTRY , vol. 4, 1993, pages 366-371, XP002010872 C.CASAS ET AL.: "Preparation of Hybrid "DNA Cleaver-Oligonucleotide" Molecules Based on a Metallotris(methylpyridiniumyl)porphyrin Motif." cited in the application

## Description

### FIELD OF THE INVENTION

This invention relates to methods for both catalytically hydrolyzing and photocleaving ribonucleic acid with a single agent, and both catalytically hydrolyzing ribonucleic acid and photocleaving deoxyribonucleic acid with a single agent.

### BACKGROUND OF THE INVENTION

Texaphyrins are aromatic pentadentate macrocyclic "expanded porphyrins" useful as MRI contrast agents, as radiosensitizers and in photodynamic therapy (PDT). They have activity for phosphate ester and RNA hydrolysis, or in RNA and DNA light-induced cleavage. Texaphyrin is considered as being an aromatic benzannulene containing both 18π- and 22π-electron delocalization pathways. See, e.g., Sessler, J.L. *et al., Accounts of Chemical Research,* **1994,** 27:43. Texaphyrin molecules absorb strongly in the tissue-transparent 730-900 nm range, and they exhibit inherent selective uptake or biolocalization in certain tissues, particularly regions such as, for example, liver, atheroma or tumor tissue. Texaphyrins and water-soluble texaphyrins, method of preparation and various uses have been described in U.S. Patents Nos. 4,935,498; 5,162,509; 5,252,720; 5,256,399; 5,272,142; 5,292,414; 5,369,101; 5,432,171; 5,439,570; 5,451,576; 5,457,183; 5,475,104 and 5,504,205; 5,599,923 (USSN 08/196,964); 5,559,207 (USSN 08/227,370); 5,587,463 (USSN 08/207,845); 5,567,687 (USSN 08/310,501); 5,594,136 (USSN 08/433,573); and PCT publications WO 90/10633, 94/29316, 95/10307, 95/21845, and 96/09315.

Texaphyrins may be coupled to site-directing molecules to form conjugates for targeted *in vivo* delivery. Site-specific ester hydrolysis of RNA with a lanthanide metal texaphyrin complex-oligonucleotide conjugate has been shown; see, Magda, D. *et al., J. Am. Chem. Soc.,* **1994,** 116:7439; and PCT publication WO 94/29316. Site-specific light-induced photocleavage of DNA with a diamagnetic metal texaphyrin complex-oligonucleotide conjugate has also been carried out; see, Magda, D. *et al., J. Am. Chem. Soc.* **1995,** 117:3629; and WO 96/09315.

While many texaphyrin metal complexes have been shown to promote the hydrolysis of phosphate ester bonds (see, WO 94/29316), and other texaphyrin metal complexes have been shown to act as catalysts for the light-induced cleavage (photocleavage) of DNA and RNA (see, WO 96/09315), it has not been previously shown that a single texaphyrin metal complex would both hydrolyze and photocleave RNA, or would both hydrolyze RNA and photocleave DNA. Such a texaphyrin complex would be very useful in practical terms, since only one metal complex would be necessary to perform a variety of functions. This is especially important in *in vivo* or *ex vivo* treatment situations where it is desirable for only a relatively small amount of material to be present. It is also desirable where the metal complex itself, such as for example where the complex is an oligonucleotide conjugate, is quite expensive or can be produced in only small quantities. Additionally, while two separate conjugates could be employed to cover both photocleavage and hydrolysis activities, respectively, the conjugates could compete with one another.

### SUMMARY OF THE INVENTION

The inventors have now discovered a particular class of texaphyrin metal complexes which both hydrolyzes and photocleaves RNA. This class of complexes also both hydrolyzes RNA and photocleaves DNA.

In particular, in one embodiment, the present invention is directed to a method for both hydrolyzing and photocleaving ribonucleic acid, the method comprising the steps of contacting the ribonucleic acid with a texaphyrin metal complex having catalytic activity for both hydrolysis and photocleavage of ribonucleic acid, incubating the ribonucleic acid and the metal complex under conditions and for a time sufficient to hydrolyze the phosphate ester bond of the ribonucleic acid, and exposing the texaphyrin metal complex to light for a time sufficient to photocleave the ribonucleic acid.

In another embodiment of the present invention, the invention is directed to a method for both hydrolyzing ribonucleic acid and photocleaving deoxyribonucleic acid, the method comprising the steps of contacting a mixture of the ribonucleic acid and the deoxyribonucleic acid with a texaphyrin metal complex having catalytic activity for both hydrolysis of a ribonucleic acid and photocleavage of deoxyribonucleic acid, incubating the mixture and the metal complex under conditions and for a time sufficient to hydrolyze the phosphate ester bond of the ribonucleic acid, and exposing the texaphyrin metal complex to light for a time sufficient to photocleave the deoxyribonucleic acid.

### DETAILED DESCRIPTION OF THE PREFERRED EMBODIMENTS

The present invention discloses the use of a single texaphyrin metal complex to perform two separate chemical reactions. Specifically, the texaphyrin metal complex has the ability to effect both hydrolysis and photocleavage reactions. In one reaction, a phosphate ester bond is cleaved by hydrolysis; that is, the cleavage is a hydrolytic reaction where a water molecule is added across an ester bond to break the bond. This hydrolytic reaction is particularly useful for cleaving the phosphate ester bonds of RNA, and is not dependent on the presence or absence of light. The second reaction, by comparison, is a photolytic cleavage reaction; that is, the texaphyrin metal complex, when irradiated in the presence of oxygen serves to produce cytotoxic materials, such as singlet oxygen, which products then cause strand damage or breakage, or "photocleavage", of DNA or RNA. Not wanting to be bound by theory, it is possible that singlet oxygen attacks a purine base, such as guanine for example, and causes depurination of the nucleic acid similar to the Maxam and Gilbert chemical cleavage of DNA. It is important to note that while strand breakage, as outlined herein, is useful for quantitating the extent of photochemically derived damage, this damage in itself (e.g., modification of a nucleotide) is known to inhibit biological function of the nucleic acid (for example, translation of RNA, phage infectivity). This photocleavage reaction is dependent on the presence of light, and in particular light in the spectral range of about 650-900 nm, preferably about 700-800 nm, and most preferably about 730-770 nm, where living tissues are relatively transparent.

The ability of a single agent to catalyze both types of reactions is especially useful in the design of therapy treatments that take advantage of both hydrolysis and photocleavage mechanisms. Thus, for example, a single texaphyrin metal complex can be targeted to a particular RNA, such as an antisense texaphyrin complex-oligonucleotide conjugate directed to a target mRNA. This metal complex-conjugate is administered to a patient, after which light is applied in proximity to the metal complex and the target mRNA to cause photocleavage of the RNA. The RNA may be a solution or a suspension of RNA or may be cellular RNA *in vitro, in vivo,* or *ex vivo.* The ability to hydrolyze and cleave RNA has important implications for the treatment of various diseases; for destruction of retroviral RNA, messenger RNA, ribosomal RNA, RNA cofactors, transfer RNA, small nuclear RNA, and small cytoplasmic RNA, thereby providing a multifactorial approach to eliminating diseased, cancerous or other unwanted cells or tissues. The texaphyrin complex is not destroyed during the photocleavage reaction, so that after the light is removed, the complex-conjugate remains to cleave, via light-independent hydrolysis, any target RNA which may remain.

In a second example, a single texaphyrin metal complex can be used in the cleavage and destruction of two different targets, one RNA-based and the other DNA-based, via hydrolytic and photocleavage action. RNA targets include those described herein. Site-specific photocleavage of DNA has important ramifications in a variety of applications. Potential particular applications include antisense applications, photocleavage affecting recombination of DNA, destruction of viral DNA, or control of gene expression at the cellular level. In contrast to using two agents, each targeting either RNA or DNA only, the use of one agent to target both RNA and DNA precludes the reduction in efficiency caused by the presence of two competing agents. Additionally, less total drug needs to be utilized to effect the desired result.

The methods of the present invention are conducted under conditions sufficient to hydrolyze the RNA and photocleave the DNA or RNA. Such conditions include physiologic conditions. This is especially useful when the texaphyrin complexes are used *in vivo* or *ex vivo* as a treatment procedure to hydrolyze RNA and also photocleave RNA or DNA.

In the practice of the present invention, the texaphyrin macrocycle to be complexed to the metal ion may be chosen from any texaphyrin molecule, including those now known and disclosed in the U.S. patents and patent applications cited herein. Representatives of texaphyrin metal complexes included within the present invention are encompassed within the following formula:

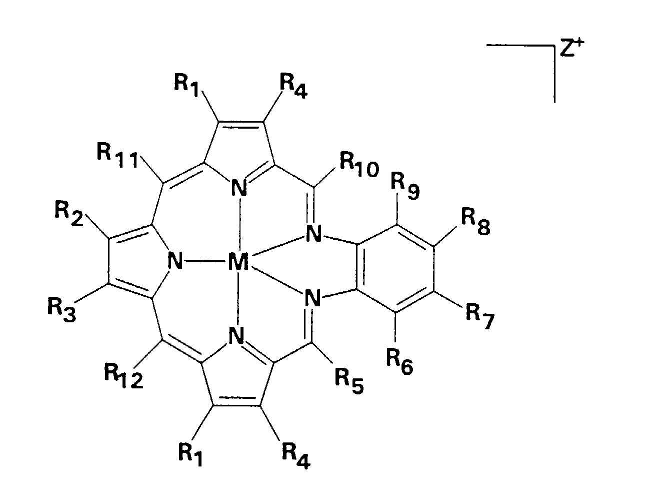

M is a divalent metal cation or a trivalent metal cation having catalytic activity for both hydrolysis of RNA and photocleavage of RNA or DNA. Metals useful in the present invention may include cadmium(II), yttrium(III), indium(III), lanthanum(III), lutetium(III), and other diamagnetic metal cations.

R₁-R₄, R₇ and R₈ are independently hydrogen, halide, hydroxyl, alkyl, alkenyl, alkynyl, aryl, haloalkyl, nitro, formyl, acyl, hydroxyalkyl, oxyalkyl, oxyhydroxyalkyl, hydroxyalkenyl, hydroxyalkynyl, saccharide, carboxy, carboxyalkyl, carboxyamide, carboxyamidealkyl, amino, aminoalkyl, a site-directing molecule, a catalytic group, or a couple that is coupled to a site-directing molecule or to a catalytic group.

R₆ and R₉ are independently selected from the groups of R₁-R₄, R₇ and R₈, with the proviso that the halide is other than iodide and the haloalkyl is other than iodoalkyl.

R₅ and R₁₀-R₁₂ are independently hydrogen, alkyl, alkenyl, alkynyl, aryl, hydroxyalkyl, oxyalkyl, oxyhydroxyalkyl, hydroxyalkenyl, hydroxyalkynyl, carboxyalkyl, carboxyamide, carboxyamidealkyl, amino, aminoalkyl, or a couple that is coupled to a saccharide, to a site-directing molecule or to a catalytic group.

The charge, Z, is an integer value less than or equal to 5. In the context of the basic macrocycle with a yttrium cation, Z is 1 or 2. However, the charge Z would be altered so as to account for the choice of metal M, the pH under consideration, and charges present on any of substituents R₁-R₁₂ and charges present on any covalently bound site-directing molecule, for example charges of the phosphate groups on an oligonucleotide. For instance, if R₁ = carboxyl and R₂-R₁₂ = alkyl and the metal M = Lu⁺³, and the solution is pH = 7 (so that R₁ = CO₂⁻), the charge Z would be zero. The charge would be negative when substituents have a sufficient number of negative charges, for example, when a substituent is an oligonucleotide. The charge would be +5, for example, when the M is Lu⁺³ and the net charge of a substituent(s) is three positive charges. Complexes described in the present invention have one or more additional ligands providing charge neutralization and/or coordinative saturation to the metal ion. Such ligands include chloride, nitrate, acetate, and hydroxide, for example.

"Alkyl" means alkyl groups, straight, branched or cyclic isomers, with generally one to fifty, preferably one to thirty, more preferably one to ten, carbon atoms.

"Alkenyl" means alkenyl groups, straight, branched or cyclic isomers, with generally two to fifty, preferably two to thirty, more preferably two to ten, carbon atoms, and with one to five or more double bonds, preferably one to five, more preferably one to three double bonds.

"Alkynyl" means alkynyl groups, straight, branched or cyclic isomers, with generally two to fifty, preferably two to thirty, more preferably two to ten, carbon atoms, and with one to five or more triple bonds, preferably one to five, more preferably one to three triple bonds.

"Hydroxyalkyl" means alcohols of alkyl groups. Preferred are hydroxyalkyl groups having one to twenty, more preferably one to ten, hydroxyls. "Hydroxyalkyl" is meant to include polyethers with one or more functional groups; diols of alkyls, with diols of C₁₋₁₀ alkyls being preferred, and diols of C₁₋₃ alkyls being more preferred; and polyethylene glycol, polypropylene glycol and polybutylene glycol as well as polyalkylene glycols containing combinations of ethylene, propylene and butylene.

"Oxyalkyl" means alkyl groups as herein described with oxygen atoms, including ether linkages. The number of repeating oxyalkyls within a substituent may be up to 200, preferably from 1 to 20, more preferably from 1 to 7, and most preferably is 2-3. A preferred oxyalkyl is O(CH₂CH₂O)ₓCH₃ where x = 1-120, preferably 1-10, and more preferably, 1-5.

"Hydroxyalkoxy" means alkyl groups as described herein having ether or ester linkages, as well as hydroxyl groups, substituted hydroxyl groups, carboxyl groups, substituted carboxyl groups or the like.

"Carboxy" groups include carboxylic acids of the alkyls described herein as well as aryl carboxylic acids such as benzoic acid. "Carboxyalkyl" means alkyl groups having hydroxyl groups, carboxyl or amide substituted ethers, ester linkages, tertiary amide linkages removed from the ether or the like. Representative examples of "carboxyamides" include primary carboxyamides (CONH₂), and secondary (CONHR') and tertiary (CONR'R") carboxyamides where each of R' and R" is a functional group as described herein. "Carboxyamidealkyl" means alkyl groups with secondary or tertiary amide linkages or the like.

Representatives of useful amines include a primary, secondary or tertiary amine of an alkyl as described hereinabove.

"Aryl" is an aromatic group whose molecules have the ring structure characteristic of benzene, naphthalene, phenanthrene, anthracene, and the like, i.e., either the 6-carbon ring of benzene or the condensed 6-carbon rings of the other aromatic derivatives. For example, an aryl group may be phenyl or naphthyl, unsubstituted or substituted with a nitro, carboxy, sulfonic acid, hydroxy, oxyalkyl or halide substituent. In this case, the substituent on the phenyl or naphthyl may be added in a synthetic step after the condensation step which forms the macrocycle.

The term "saccharide" includes oxidized, reduced or substituted saccharide; hexoses such as D-glucose, D-mannose or D-galactose; pentoses such as D-ribose or D-arabinose; ketoses such as D-ribulose or D-fructose; disaccharides such as sucrose, lactose, or maltose; derivatives such as acetals, amines, and phosphorylated sugars; oligosaccharides; as well as open chain forms of various sugars, and the like. Examples of amine-derivatized sugars are galactosamine, glucosamine, and sialic acid.

Hydrolytic cleavage of phosphate ester bonds, and particularly of RNA, by texaphyrin complexes may be enhanced by additional catalytic groups appended to the texaphyrin complex or to a texaphyrin complex-site directing molecule conjugate. The term "catalytic group" means a chemical functional group that assists catalysis by acting as a general acid, Brønsted acid, general base, Brønsted base, nucleophile, or any other means by which the activation barrier to reaction is lowered or the ground state energy of the substrate is increased. Exemplary catalytic groups contemplated include imidazole; guanidine; substituted saccharides such as D-glucosamine, D-mannosamine, D-galactosamine, D-glucamine and the like; amino acids such as L-histidine and L-arginine; derivatives of amino acids such as histamine; polymers of amino acids such as poly-L-lysine, (LysAla)ₙ, (LysLeuAla)ₙ where n is from 1-30 or preferably 1-10 or more preferably 2-7; derivatives thereof; and texaphyrin metal complexes. The term "appended to the texaphyrin complex-site directing molecule conjugate" means that the catalytic groups are attached either directly to the texaphyrin metal complex or to the texaphyrin complex via a linker or couple of variable length, or are attached to the ligand portion of a texaphyrin complex-ligand conjugate either with or without a linker or couple of variable length.

As used herein, a "site-directing molecule" means a molecule having affinity and specificity for a particular target site. Exemplary site-directing molecules useful herein include, but are not limited to, polydeoxyribonucleotides, oligodeoxyribonucleotides, polyribonucleotide analogs, oligoribonucleotide analogs, polyamides including peptides having affinity for a biological receptor and proteins such as antibodies, steroids and steroid derivatives, hormones such as estradiol or histamine, hormone mimics such as morphine, and further macrocycles such as sapphyrins and rubyrins. It is understood that the terms "nucleotide", "polynucleotide" and "oligonucleotide", as used herein and in the appended claims, refer to both naturally-occurring and synthetic nucleotides, poly-and oligonucleotides and to analogs and derivatives thereof such as methylphosphonates, phosphotriesters, phosphorothioates, phosphoramidates and the like. Deoxyribonucleotides, deoxyribonucleotide analogs and ribonucleotide analogs are contemplated as site-directing molecules in the present invention.

Oligonucleotides have several advantages over traditional drugs, notably their exquisite specificity to target sites and their ease of design. Oligonucleotides may be derivatized at the bases, the sugars, the ends of the chains, or at the phosphate groups of the backbone to promote *in vivo* stability. CpG sequences may be derivatized to minimize degradation; derivatization may be alkylation, and is preferably methylation. Modifications of the phosphate groups are preferred in one embodiment of the invention since phosphate linkages are sensitive to nuclease activity. Preferred derivatives are the methylphosphonates, phosphotriesters, phosphorothioates, and phosphoramidates. Derivatives may also contain alternating phosphorothioate and unmodified linkages, or alternating methylphosphonate and unmodified linkages, or alternating phosphorothioate and methylphosphonate linkages. Additionally, the phosphate linkages may be completely substituted with non-phosphate linkages such as amide linkages. Appendages to the ends of the oligonucleotide chains also provide exonuclease resistance. The 5' or 3' end may be derivatized or "capped" with a phosphoramidate linkage, an inverted nucleotide conjugated to the oligonucleotide via a 3'-3' linkage, an aminoacridine residue, or poly-L-lysine.

Sugar modifications may include groups, such as halo, alkyl, alkenyl or alkoxy groups, attached to an oxygen of a ribose moiety in a ribonucleotide. In a preferred embodiment, the group will be attached to the 2' oxygen of the ribose. In particular, halogen moieties such as fluoro may be used. The alkoxy group may be methoxy, ethoxy or propoxy. The alkenyl group is preferably allyl. The alkyl group is preferably a methyl group and the methyl group is attached to the 2' oxygen of the ribose. Other alkyl groups may be ethyl or propyl.

The term "texaphyrin-oligonucleotide conjugate" means that an oligonucleotide is attached to the texaphyrin in a 5' linkage, a 3' linkage, or both types of linkages that would allow the texaphyrin to be an internal residue in the conjugate. It can also refer to a texaphyrin that is linked to an internal base of the oligonucleotide. The oligonucleotide or other site-directing molecule may be attached either directly to the texaphyrin or to the texaphyrin via a linker or a couple of variable length. During catalysis, for example, the texaphyrin portion of a texaphyrin metal complex-oligonucleotide conjugate is placed in the vicinity of the substrate upon binding of the oligonucleotide to the targeted nucleic acid substrate.

The term "a peptide having affinity for a biological receptor" means that upon contacting the peptide with the biological receptor, for example, under appropriate conditions of ionic strength, temperature, pH and the like, specific binding will occur. The interaction may occur due to specific electrostatic, hydrophobic, entropic or other interaction of certain amino acid or glycolytic residues of the peptide with specific amino acid or glycolytic residues of the receptor to form a stable complex under the conditions effective to promote the interaction. The interaction may alter the three dimensional conformation and the function or activity of either or both the peptide and the receptor involved in the interaction. A peptide having affinity for a biological receptor may include an endorphin, an enkephalin, a growth factor, e.g. epidermal growth factor, poly-L-lysine, a hormone, a peptide region of a protein and the like. Representative examples of useful polypeptides include both naturally occurring and synthetic polypeptides (e.g., insulin, ribonuclease, and β-endorphin).

Representative examples of useful steroids include any of the steroid hormones of the following five categories: progestagens such as progesterone, glucocorticoids such as cortisol, mineralocorticoids such as aldosterone, androgens such as testosterone or androstenedione, and estrogens such as estrone or estradiol.

A conjugated group having site specificity or catalytic activity may be covalently coupled to a texaphyrin directly on the macrocycle ring or through various couples. A couple may be described as a linker, i.e., the covalent product formed by reaction of a reactive group designed to attach covalently another molecule at a distance from the texaphyrin macrocycle. Exemplary linkers or couples are amides, amine, disulfide, thioether, ether, ester, or phosphate covalent bonds. In preferred embodiments, conjugates and appended groups are covalently bonded to the texaphyrin via a carbon-carbon, a carbon-nitrogen, a carbon-sulfur, or a carbon-oxygen bond, more preferred being a carbon-oxygen or a carbon-nitrogen bond.

In the practice of the present invention, in a preferred embodiment at least one of R₁-R₁₂ is a site-directing molecule or is a couple to a site-directing molecule. Also presently preferred are those compounds where R₁ is hydroxyalkyl and R₂, R₃, and R₄ are alkyl. R₇ and R₈ may be hydroxyalkoxy or oxyalkyl. Alternatively, R₃, R₇ or R₈ may be a site-directing molecule or a couple to a site-directing molecule. Preferred site-directing molecules are a hormone or an oligonucleotide, more preferably an oligonucleotide. The oligonucleotide may be a deoxyribonucleotide, a deoxyribonucleotide derivative or analog, or a ribonucleotide analog. A presently preferred ribonucleotide analog, for example, has alkyl groups, more preferably methyl groups, on the 2' oxygen of the ribose. A presently preferred deoxyribonucleotide analog is a phosphorothioate or a phosphoramidate.

In a further preferred texaphyrin complex of the present invention, R₁ is (CH₂)₂CH₂OH, R₂ and R₃ are CH₂CH₃, R₄ is CH₃, and R₇ and R₈ are OCH₂CH₂CH₂OH or R₇ and R₈ are O(CH₂CH₂O)ₜCH₂CH₂OR' where t is zero to 10, preferably zero to 3, and R' is H or CH₃. Alternatively, R₇ is a site-directing molecule or a couple thereto, preferably an oligonucleotide or a couple thereto, more preferably O(CH₂)ₙCO-oligonucleotide where n is 1-7 and preferably 1-3. Where R₇ is a site-directing molecule or a couple thereto, R₈ may be H, OCH₃ or one of the previously listed preferred substituents.

A presently preferred metal is yttrium(III).

Water-soluble texaphyrins are often preferred for the applications described herein, particularly when *in vivo* administration and treatment is contemplated. "Water-soluble" means soluble in aqueous fluids to about 1 mM or better. Such characteristics allow these texaphyrins to be useful in a biological environment. Improved water solubility can be achieved by, for example, substituents chosen from saccharides or hydroxylated substituents.

In presently preferred texaphyrin compounds, **R**_{**1**}**-R**_{**4**}**, R**_{**7**}, and **R**_{**8**} are as in Table 1 for texaphyrins A1-A22, **R**_{**5**}**, R**_{**6**}, and **R**_{**9**}**-R**_{**12**} are H, and M is as defined hereinabove. However, while the above-described texaphyrins are presently preferred compounds for use in the present invention, the invention is not limited thereto and any texaphyrin complex that has activity both as a hydrolyzing agent for RNA and as a photocleaving agent for RNA or DNA may be useful in the practice of the invention.

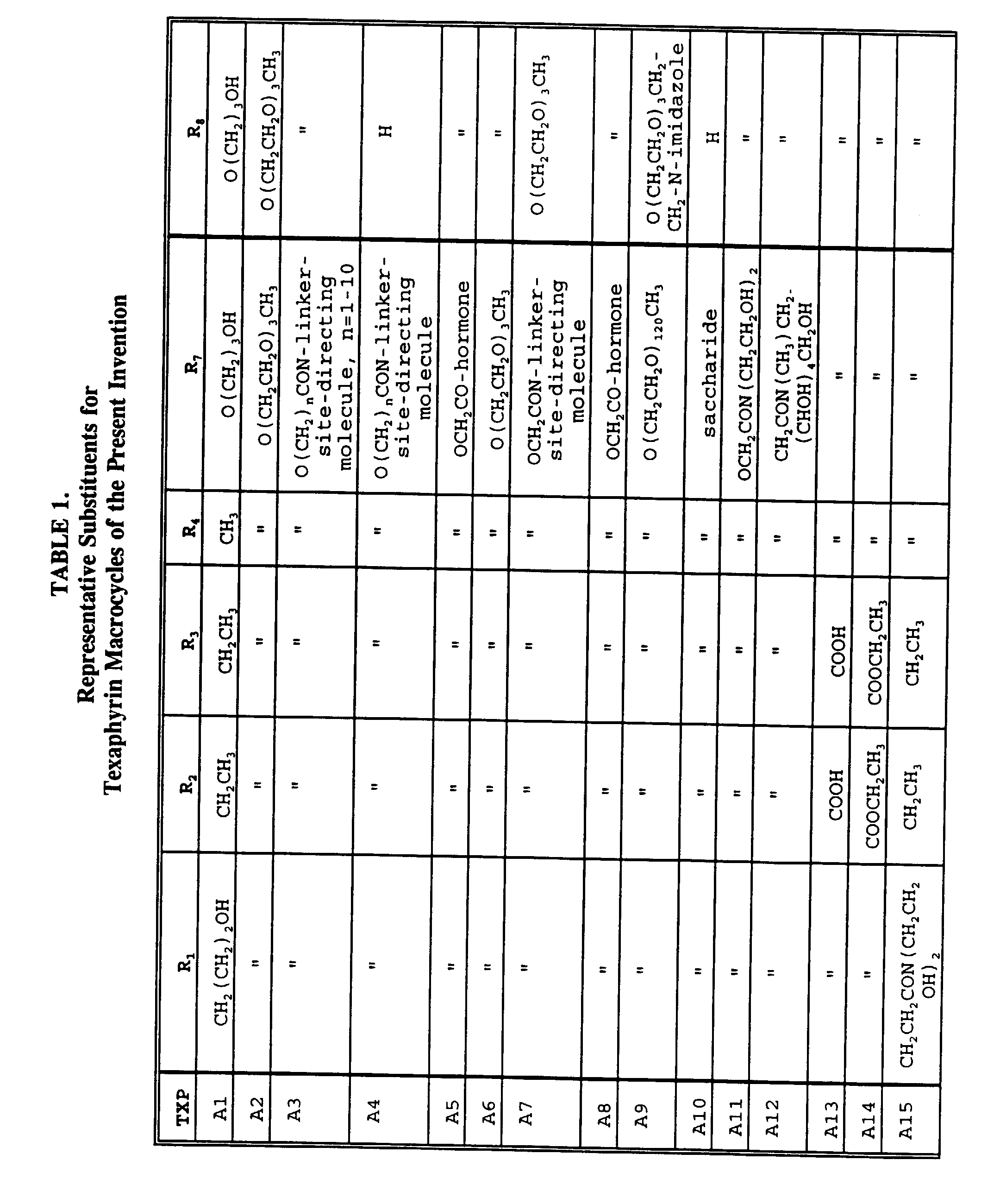

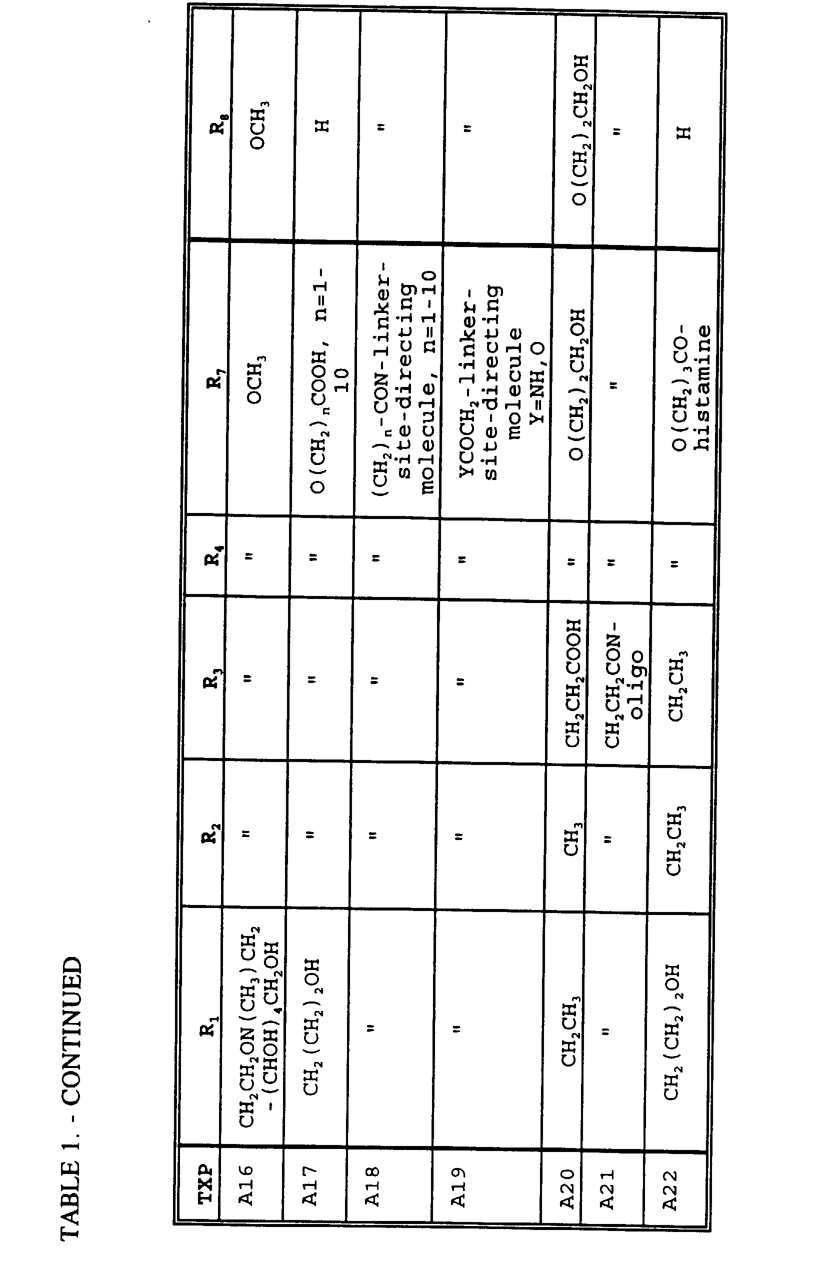

The texaphyrin metal complexes of the present invention can be synthesized by procedures as described in the patents and applications cited herein. For example, a nonaromatic texaphyrin of structure I is mixed together with a metal salt, a Brønsted base and an oxidant, in an organic solvent, and the mixture is allowed to react to form an aromatic texaphyrin metal complex. A preferred means is to stir the mixture at ambient temperature or to heat the mixture at reflux for at least two hours.

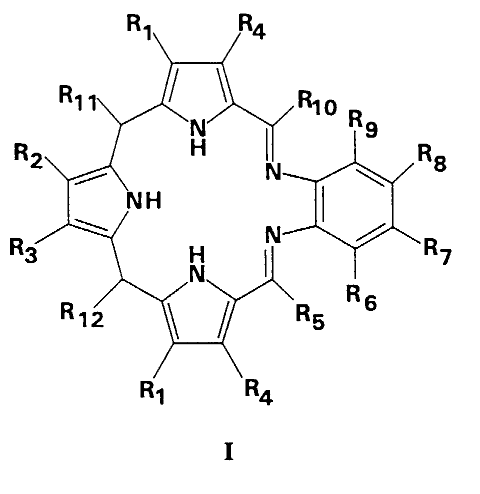

The nonaromatic texaphyrin **I** is conveniently produced by condensation of a tripyrrane aldehyde or ketone having structure A and a substituted *ortho*phenylenediamine B:

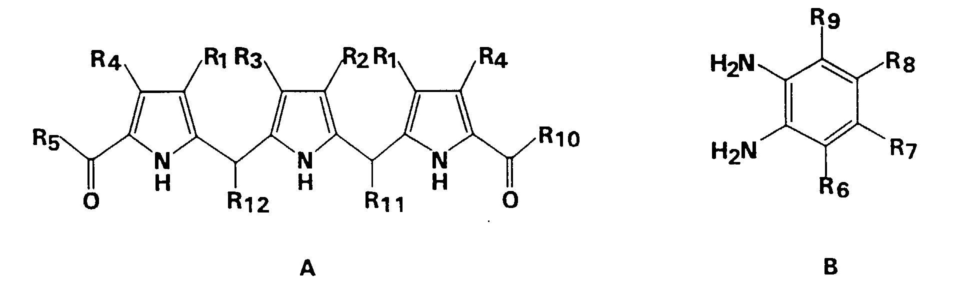

One skilled in the art of organic synthesis in light of the present disclosure and the disclosures in the patents, applications and publications cited herein could extend and refine the above basic synthetic chemistry to produce texaphyrins having various substituents. For example, polyether-linked polyhydroxylated groups, saccharide substitutions in which the saccharide is appended via an acetal-like glycosidic linkage, an oligosaccharide or a polysaccharide may be similarly linked to a texaphyrin. A doubly carboxylated texaphyrin in which the carboxyl groups are linked to the texaphyrin core via aryl ethers or functionalized alkyl substituents could be converted to various esterified products wherein the ester linkages serve to append further hydroxyl-containing substituents. Polyhydroxylated texaphyrin derivatives may be synthesized via the use of secondary amide linkages. Saccharide moieties may be appended via amide bonds. Polyhydroxylated texaphyrin derivatives containing branched polyhydroxyl (polyol) subunits may be appended to the texaphyrin core via aryl ethers or ester linkages.

Treatment of carboxylated texaphyrins with thionyl chloride or *p*-nitrophenol acetate would generate activated acyl species suitable for attachment to monoclonal antibodies or other biomolecules of interest. Standard in situ coupling methods (e.g., 1,1'-carbonyldiimidazole) could be used to effect the conjugation.

The selectivity of the texaphyrins may be enhanced by covalently linking oligonucleotides onto the periphery of the macrocycle. Amides, ethers and thioethers are representative of linkages which may be used for this purpose. Oligonucleotides functionalized with amines at the 5'-end, the 3'-end, or internally at sugar or base residues may be modified post-synthetically with an activated carboxylic ester derivative of the texaphyrin complex. Alternatively, oligonucleotide analogs containing one or more thiophosphate or thiol groups may be selectively alkylated at the sulfur atom(s) with an alkyl halide derivative of the texaphyrin complex. The resultant oligonucleotide-complex conjugates may be designed so as to provide optimal catalytic interaction between a target nucleic acid and the bound texaphyrin. The oligonucleotide may be large enough to bind probably at least about 8 nucleotides of complementary nucleic acid. Specific methods for preparing texaphyrin-oligonucleotide conjugates are disclosed in WO 94/29316.

The use of texaphyrin complexes to hydrolyze RNA and also photocleave RNA or DNA *in vivo* as a treatment procedure relies on the effective localization of the complex to the site of desired cleavage. A site of desired cleavage may be a position novel to undesired organisms in terms of health care. A site of desired cleavage may be a DNA or an RNA encoding a product deleterious to the host or may be a normal DNA or RNA that is deleterious in some way. Treating native RNA or DNA with a texaphyrin oligonucleotide conjugate results in the texaphyrin conjugate binding to a complementary RNA or DNA sequence via the appended oligonucleotide. The texaphyrin complex then cleaves the RNA or DNA proximal to this specific site. The binding of a conjugate to a DNA double helix will form a triple helix which has sufficient stability for effective cleavage to occur.

The texaphyrin-oligonucleotide conjugates may be useful for inhibiting the expression of a gene in an animal or in a particular tissue of an animal. They may also be useful in a method for targeted intracellular mRNA hydrolysis and photocleavage, or for targeted intracellular mRNA hydrolysis and DNA photocleavage. The texaphyrin-oligonucleotide conjugates and present method of hydrolysis and photocleavage would have immediate applications for anti-viral and anti-bacterial therapy as well as cancers (an oligonucleotide complementary to an oncogene, for example) and inflammatory responses that are caused by the overexpression of certain proteins.

An exemplary method for delivering texaphyrin-oligonucleotide conjugates into a cell is the use of texaphyrin-oligonucleotide-glycoconjugates for carrying oligonucleotides specific for targeted sequences. Texaphyrin-oligonucleotide conjugates linked through a disulfide bridge to a glycoconjugate could be significantly more effective in reaching a target site than the corresponding free oligonucleotide. Poly-L-lysine can be substituted by three components: an oligonucleotide as a recognition signal, a therapeutic texaphyrin-metal complex, and gluconoic acid as a neutralizing and solubilizing agent. This type of neutral, highly water-soluble glycosylated polymer may be an efficient carrier to deliver drugs into cells.

For the above-described uses, texaphyrins are provided as pharmaceutical preparations. A pharmaceutical preparation of a texaphyrin may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining a texaphyrin of the present invention and the pharmaceutically acceptable carriers are then easily administered in a variety of dosage forms. Administration may be intravenous, intraperitoneal, parenteral, intramuscular, subcutaneous, oral, or topical, with topical and intravenous administration being preferred, and intravenous being more preferred.

Solutions of the texaphyrin in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. Topical creams, emulsions, solutions, and the like are contemplated for applications to surface areas of the body. Topical application may also be by iontophoresis.

For antisense applications, excipients and preservatives that preserve oligonucleotide stability are chosen. The highly negatively charged phosphate or sulfur groups of the backbone of the oligonucleotide may be irritating to epithelial or other surface cells. Counterions may be used for formulation purposes to prevent irritation.

Pharmaceutical forms include sterile aqueous solutions or dispersions and sterile powders for the extemporaneous preparation of sterile injectable solutions or dispersions. In all cases the form must be sterile and must be fluid to the extent that easy use with a syringe exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars such as mannitol or dextrose or sodium chloride. A more preferable isotonic agent is a mannitol solution of about 2-8% concentration, and, most preferably, of about 5% concentration. Prolonged absorption of the injectable compositions can be brought about by the use in the compositions of agents delaying absorption, for example, aluminum monostearate and gelatin.

Sterile solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

As used herein, "pharmaceutically acceptable carrier" includes any and all solvents, dispersion media, coatings, permeation enhancers, antibacterial and antifungal agents, isotonic and absorption delaying agents and the like. The use of such media and agents for pharmaceutically active substances is well known in the art. Except insofar as any conventional media or agent is incompatible with the active ingredient, its use in the therapeutic compositions is contemplated. Supplementary active ingredients can also be incorporated into the compositions.

A reliable assay for RNA or DNA photocleavage potency of a texaphyrin-oligonucleotide conjugate is an assay for cleavage of a complementary ribonucleic acid or deoxyribonucleic acid, respectively. A reliable assay for RNA hydrolysis is an assay for hydrolysis of a complementary ribonucleic acid as described in WO 94/29316. Cleavage by a conjugate would demonstrate that the conjugate has the intended potency and activity.

The following example is an illustration of the practice of the present invention, and is intended neither to define nor to limit the scope of the invention in any manner.

### EXAMPLE 1

The present example illustrates site-specific, light-independent hydrolysis of RNA and site-specific light-dependent photocleavage of DNA by yttrium(III) texaphyrin-DNA oligonucleotide conjugates. The RNA hydrolysis properties of the YTx conjugates are compared with those of analogous paramagnetic Dy(III)Tx conjugates, and the DNA photocleavage properties of the YTx conjugates are compared with those of analogous diamagnetic Lu(III)Tx conjugates.

The texaphyrin-oligonucleotide conjugate in each case had the following formula, where M is yttrium, dysprosium or lutetium:

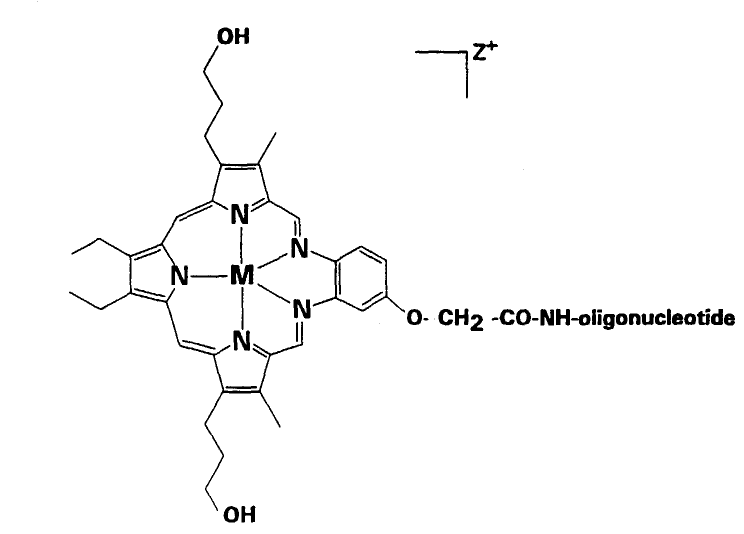

Methods for the preparation of the above texaphyrins, as well as those of other texaphyrins and texaphyrin-oligonucleotide conjugates, are described in WO 94/29316, the disclosure of which is incorporated herein by reference. Scheme A shows yttrium(III) texaphyrin (YTx) DNA conjugates, dysprosium(III) texaphyrin (DyTx) DNA conjugates and lutetium(III) texaphyrin (LuTx) DNA conjugates of the present example, and complementary 5'-³²P-labeled DNA 36-mers and 5'-³²P-

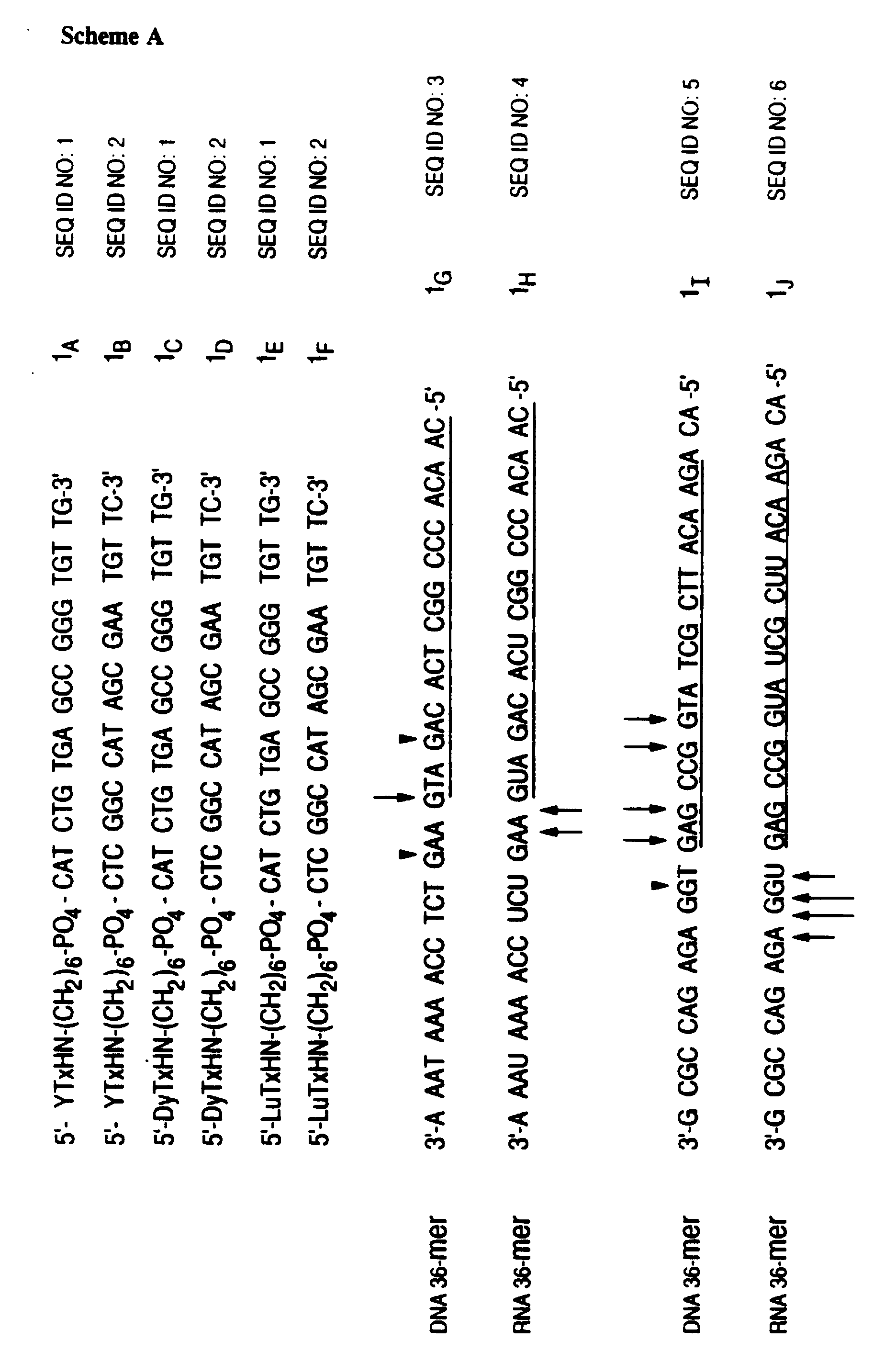

labeled RNA 36-mers. The DNA sequences used in the conjugates were purchased from Keystone Labs, Menlo Park, CA; and the RNA sequences were purchased from Promega Corp., Madison, WI.

Reaction mixtures were prepared by adding YTx conjugate **1**_{**A**} or **1**_{**B**}, DyTx conjugate **1**_{**C**} or **1**_{**D**}, or LuTx conjugate **1**_{**E**} or **1**_{**F**} to solutions made from ca. 100,000 cpm of 5'-³²P-labeled DNA 36-mer **1**_{**G**} or **1**_{**H**} or 5'-³²P-labeled RNA 36-mer **1**_{**I**} or **1**_{**J**} mixed with 4X buffer (5µl (µL)), carrier DNA (1µl (µL)) and water to produce a final volume of 20 µl (µL). Final conjugate concentration was 50 nM. The 4X buffer was 400 mM NaCl, 200 mM HEPES, pH 7.5, 100 µM EDTA. All reactions were heated for 4 minutes at 58°C and allowed to cool slowly to ambient temperature.

RNA hydrolysis reactions were covered and incubated at 37°C for 22 h(hr). DNA photocleavage reactions were irradiated for 15 min. at ambient temperature using a dye laser (Coherent; Palo Alto, CA) tuned to 732 nm using a power density of 150 mW/cm².

At the end of the reaction time, the reaction samples containing radiolabeled DNA were precipitated with ethanol, then were dissolved in 10% aqueous piperidine solution (50 µl (µL)) and heated to 90°C for 30 min. Water (500 µl (µL)) was added to the DNA samples, which were then dried on a Speedvac. The samples from the RNA hydrolysis reactions were precipitated with ethanol using standard methods. All samples were resuspended in 50% formamide loading buffer, denatured at 60°C for 3 min., and analyzed by electrophoresis on a 20% denaturing polyacrylamide gel.

The autoradiograph indicated substantial cleavages only in those lanes which contained the appropriate complementary 20-mer YTx, DyTx, or LuTx conjugate. All cleavages occurred near the expected location of the YTx, DyTx, or LuTx complex upon hybridization of the conjugates with their targets. The cleavage locations on the 36-mers are indicated by arrows in Scheme A. The YTx- and LuTx-mediated DNA cleavage bands co-migrated with bands generated by dimethylsulfate in the guanine-specific sequencing lanes. The cleavage patterns generated by the YTx and DyTx conjugates on RNA were similar. Cleavage patterns generated by the YTx and LuTx conjugates on DNA were essentially identical. The total efficiency of RNA hydrolysis by the YTx conjugates ranged from 25-35 %. The total efficiency of DNA photocleavage by the YTx conjugates ranged from 50-60%.

These observations are consistent with a model whereby hybridization of the YTx conjugates to complementary sequences of DNA effects site-specific photomodification of guanine residues, and results in site-specific photocleavage upon workup under basic conditions. By contrast, in a dark reaction, the YTx conjugates effect site-specific hydrolysis of complementary RNA targets. Thus, the YTx-DNA conjugates were able to damage nucleic acids by two distinct mechanisms, hydrolysis and photocleavage. Further, as conjugates containing LuTx, a diamagnetic metallotexaphyrin analogous to YTx, have previously been shown to effect site-specific photocleavage of both DNA and RNA targets, the above data imply that YTx conjugates would also effect site-specific photocleavage of RNA targets.

### EXAMPLE 2

The present inventors have demonstrated that texaphyrin-oligonucleotide conjugates are taken up by eukaryotic cells, as observed by fluorescent localization.

HL-60 cells (human promyelocytic leukemia cell line) were incubated with a solution (5µmol final conc.) of a texaphyrin-oligonucleotide conjugate complexed with either a Y(III) metal ion or a Lu(III) metal ion (where the oligonucleotide is a phosphorothioate with 15 bases). The cells were incubated for a minimum of 10 min. and up to about 60 min., after which the cells were washed. Fluorescence was measured with a confocal argon laser, which excites at 488 nm. To view the fluorescence created by the texaphyrin, a cut-off filter was used to eliminate wavelengths below 700 nm. The resulting fluorescence images showed diffuse cytoplasmic fluorescence with some evidence of local "hot spots" of concentrated fluorescence.

## Claims

1. A method of both hydrolyzing and photocleaving ribonucleic acid, the method comprising:
contacting the ribonucleic acid with a texaphyrin metal complex having catalytic activity for both hydrolysis and photocleavage of ribonucleic acid,
incubating the ribonucleic acid and the metal complex under conditions and for a time sufficient to hydrolyze the phosphate ester bond of the ribonucleic acid, and
exposing the texaphyrin metal complex to light for a time sufficient to photocleave the ribonucleic acid.

2. A method of both hydrolyzing ribonucleic acid and photocleaving deoxyribonucleic acid, the method comprising:
contacting a mixture of the ribonucleic acid and the deoxyribonucleic acid with a texaphyrin metal complex having catalytic activity for both hydrolysis of ribonucleic acid and photocleavage of deoxyribonucleic acid,
incubating the mixture and the metal complex under conditions and for a time sufficient to hydrolyze the phosphate ester bond of the ribonucleic acid, and
exposing the texaphyrin metal complex to light for a time sufficient to photocleave the deoxyribonucleic acid.

3. The method of claim 1 or 2 wherein the texaphyrin metal complex is a texaphyrin-diamagnetic metal complex.

4. The method of claim 1 or 2 wherein the texaphyrin metal complex is a yttrium-texaphyrin complex.

5. The method of claim 1 or 2 wherein the light has a wavelength range of about 700-800 nm.

6. The method of claim 1 or 2 wherein the step of exposing the texaphyrin metal complex to light is carried out in the presence of oxygen.

7. The method of claim 1 wherein the texaphyrin metal complex has the formula: 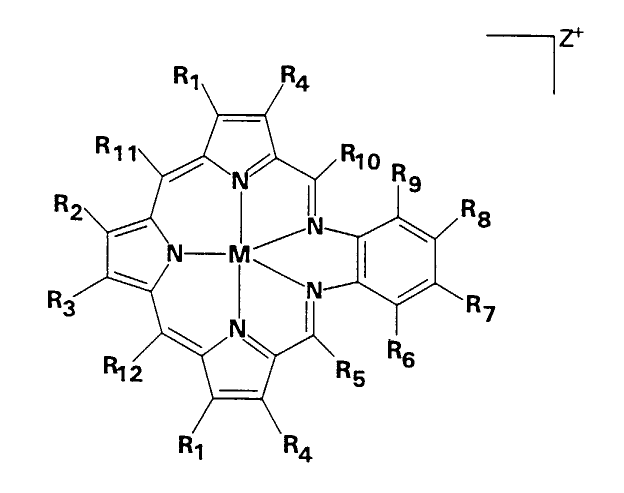 wherein,
M is a divalent metal cation or a trivalent metal cation having catalytic activity for both hydrolysis and photocleavage of ribonucleic acid;
R₁-R₄, R₇ and R₈ are independently hydrogen, halide, hydroxyl, alkyl, alkenyl, alkynyl, aryl, haloalkyl, nitro, formyl, acyl, hydroxyalkyl, oxyalkyl, oxyhydroxyalkyl, hydroxyalkenyl, hydroxyalkynyl, saccharide, carboxy, carboxyalkyl, carboxyamide, carboxyamidealkyl, amino, aminoalkyl, a site-directing molecule, a catalytic group, or a couple to a site-directing molecule or to a catalytic group;
R₆ and R₉ are independently selected from the groups of R₁-R₄, R₇ and R₈, with the proviso that the halide is other than iodide and the haloalkyl is other than iodoalkyl;
R₅ and R₁₀-R₁₂ are independently hydrogen, alkyl, alkenyl, alkynyl, aryl, hydroxyalkyl, oxyalkyl, oxyhydroxyalkyl, hydroxyalkenyl, hydroxyalkynyl, carboxyalkyl, carboxyamide, carboxyamidealkyl, amino, aminoalkyl, or a couple to a saccharide, to a site-directing molecule or to a catalytic group; and
Z is an integer value less than or equal to 5.

8. The method of claim 2 wherein the texaphyrin metal complex has the formula: 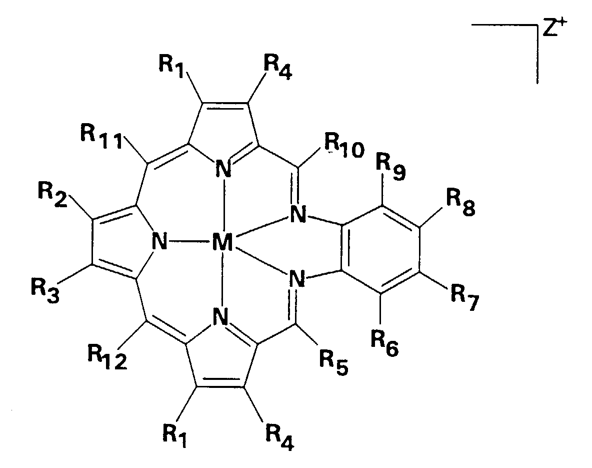 wherein,
M is a divalent metal cation or a trivalent metal cation having catalytic activity for both hydrolysis of ribonucleic acid and photocleavage of deoxyribonucleic acid;
R₁-R₄, R₇ and R₈ are independently hydrogen, halide, hydroxyl, alkyl, alkenyl, alkynyl, aryl, haloalkyl, nitro, formyl, acyl, hydroxyalkyl, oxyalkyl, oxyhydroxyalkyl, hydroxyalkenyl, hydroxyalkynyl, saccharide, carboxy, carboxyalkyl, carboxyamide, carboxyamidealkyl, amino, aminoalkyl, a site-directing molecule, a catalytic group, or a couple to a site-directing molecule or to a catalytic group;
R₆ and R₉ are independently selected from the groups of R₁-R₄, R₇ and R₈, with the proviso that the halide is other than iodide and the haloalkyl is other than iodoalkyl;
R₅ and R₁₀-R₁₂ are independently hydrogen, alkyl, alkenyl, alkynyl, aryl, hydroxyalkyl, oxyalkyl, oxyhydroxyalkyl, hydroxyalkenyl, hydroxyalkynyl, carboxyalkyl, carboxyamide, carboxyamidealkyl, amino, aminoalkyl, or a couple to a saccharide, to a site-directing molecule or to a catalytic group; and
Z is an integer value less than or equal to 5.

9. The method of claim 7 or 8 wherein M is yttrium(III).

10. The method of claim 9 wherein R₁ is (CH₂)₂CH₂OH; R₂ and R₃ are CH₂CH₃; R₄ is CH₃; R₅, R₆, R₈, R₉, R₁₀, R₁₁, and R₁₂ are H; and R₇ is OCH₂CONH-oligonucleotide.

11. The method of claim 7 or 8 wherein at least one of R₁-R₁₂ is a site-directing molecule, a catalytic group, a couple to a site-directing molecule or a couple to a catalytic group.

12. The method of claim 11 wherein the catalytic group is imidazole, guanidine, an amino acid, an amino acid derivative, a polyamino acid, an amine-substituted saccharide or a texaphyrin metal complex.

13. The method of claim 11 wherein the site-directing molecule is an oligonucleotide, a hormone, an antibody, a peptide having affinity for a biological receptor, or a sapphyrin molecule.

14. The method of claim 7 wherein at least one of R₁-R₁₂ is a site-directing molecule, and the site-directing molecule is an oligonucleotide, which oligonucleotide has binding affinity for the ribonucleic acid.

15. The method of claim 13 wherein the oligonucleotide is a derivatized oligonucleotide or an oligonucleotide analog.

16. The method of claim 15 wherein the derivatized oligonucleotide is selected from the group consisting of methylphosphonates, phosphotriesters, phosphorothioates, and phosphoramidates.

17. The method of claim 15 wherein the derivatized oligonucleotide is 2'-O-alkyl oligoribonucleotide.

18. The method of claim 13 wherein the oligonucleotide is an antisense oligonucleotide.

19. Use of a texaphyrin metal complex having catalytic activity for both hydrolysis and photocleavage of ribonucleic acid in the preparation of a pharmaceutical composition for use in both hydrolyzing and photocleaving ribonucleic acid.

20. The use of claim 19 wherein the texaphyrin metal complex has the formula: 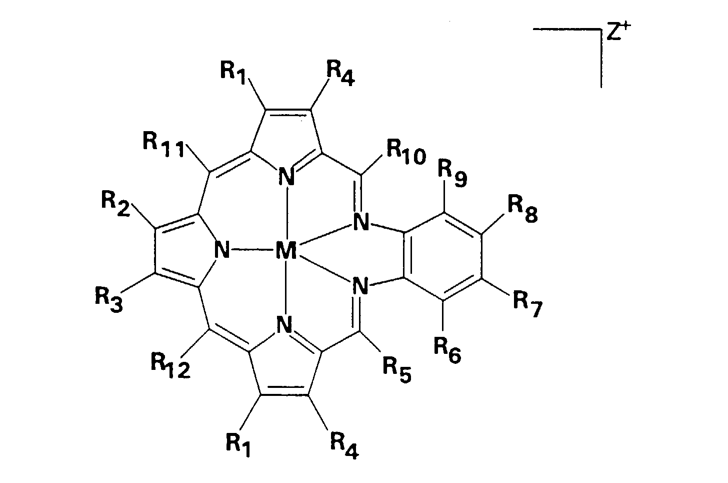 wherein,
M is a divalent metal cation or a trivalent metal cation having catalytic activity for both hydrolysis and photocleavage of the ribonucleic acid;
R₁-R₄, R₇ and R₈ are independently hydrogen, halide, hydroxyl, alkyl, alkenyl, alkynyl, aryl, haloalkyl, nitro, formyl, acyl, hydroxyalkyl, oxyalkyl, oxyhydroxyalkyl, hydroxyalkenyl, hydroxyalkynyl, saccharide, carboxy, carboxyalkyl, carboxyamide, carboxyamidealkyl, amino, aminoalkyl, a site-directing molecule, a catalytic group, or a couple to a site-directing molecule or to a catalytic group;
R₆ and R₉ are independently selected from the groups of R₁-R₄, R₇ and R₈, with the proviso that the halide is other than iodide and the haloalkyl is other than iodoalkyl;
R₅ and R₁₀-R₁₂ are independently hydrogen, alkyl, alkenyl, alkynyl, aryl, hydroxyalkyl, oxyalkyl, oxyhydroxyalkyl, hydroxyalkenyl, hydroxyalkynyl, carboxyalkyl, carboxyamide, carboxyamidealkyl, amino, aminoalkyl, or a couple to a saccharide, to a site-directing molecule or to a catalytic group; and
Z is an integer value less than or equal to 5.

21. Use of a texaphyrin metal complex having catalytic activity for both hydrolysis of ribonucleic acid and photocleavage of deoxyribonucleic acid in the preparation of a pharmaceutical composition for use in both hydrolyzing ribonucleic acid and photocleaving deoxyribonucleic acid.

22. The use of claim 21 wherein the texaphyrin metal complex has the formula: 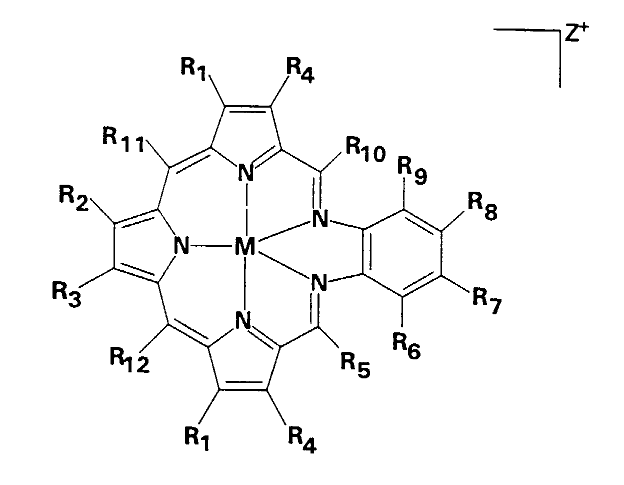 wherein,
M is a divalent metal cation or a trivalent metal cation having catalytic activity for both hydrolysis of ribonucleic acid and photocleavage of deoxyribonucleic acid;
R₁-R₄, R₇ and R₈ are independently hydrogen, halide, hydroxyl, alkyl, alkenyl, alkynyl, aryl, haloalkyl, nitro, formyl, acyl, hydroxyalkyl, oxyalkyl, oxyhydroxyalkyl, hydroxyalkenyl, hydroxyalkynyl, saccharide, carboxy, carboxyalkyl, carboxyamide, carboxyamidealkyl, amino, aminoalkyl, a site-directing molecule, a catalytic group, or a couple to a site-directing molecule or to a catalytic group;
R₆ and R₉ are independently selected from the groups of R₁-R₄, R₇ and R₈, with the proviso that the halide is other than iodide and the haloalkyl is other than iodoalkyl;
R₅ and R₁₀₋R₁₂ are independently hydrogen, alkyl, alkenyl, alkynyl, aryl, hydroxyalkyl, oxyalkyl, oxyhydroxyalkyl, hydroxyalkenyl, hydroxyalkynyl, carboxyalkyl, carboxyamide, carboxyamidealkyl, amino, aminoalkyl, or a couple to a saccharide, to a site-directing molecule or to a catalytic group; and
Z is an integer value less than or equal to 5.

23. The use of claim 20 or 22 wherein M is yttrium.

24. The use of claim 23 wherein R₁ is (CH₂)₂CH₂OH; R₂ and R₃ are CH₂CH₃; R₄ is CH₃; R₅, R₆, R₈, R₉, R₁₀, R₁₁, and R₁₂ are H; and R₇ is OCH₂CONH-oligonucleotide.

## Patentansprüche

1. Verfahren zur Hydrolysierung und Photospaltung von Ribonucleinsäure, mit den Schritten:
Kontaktherstellung der Ribonucleinsäure mit einem Texa-phyrin-Metallkomplex mit katalytischer Wirkung für die Hydrolyse und Photospaltung von Ribonucleinsäure,
Inkubation der Ribonucleinsäure und des Metallkomplexes unter Bedingungen und für eine zeit, die ausreichen, um die Phosphatesterbindung der Ribonucleinsäure zu hydrolysieren, und
Belichtung des Texaphyrin-Metallkomplexes für eine Zeit, die für die Photospaltung der Ribonucleinsäure ausreicht.

2. Verfahren zur Hydrolyse von Ribonucleinsäure und Photospaltung von Desoxyribonucleinsäure, mit den Schritten:
Kontaktherstellung einer Mischung aus Ribonucleinsäure und Desoxyribonucleinsäure mit einem Texaphyrin-Metallkomplex mit katalytischer Wirkung für die Hydrolyse der Ribonucleinsäure und Photospaltung der Desoxyribonucleinsäure,
Inkubation der Mischung und des Metallkomplexes unter Bedingungen und für eine Zeit, die ausreichen, um die Phosphatesterbindung der Ribonucleinsäure zu hydrolysieren, und
Belichtung des Texaphyrin-Metallkomplexes für eine Zeit, die für die Photospaltung der Desoxyribonucleinsäure ausreicht.

3. Verfahren nach Anspruch 1 oder 2, worin der Texaphyrin-Metallkomplex ein Texaphyrin-diamagnetischer Metallkomplex ist.

4. Verfahren nach Anspruch 1 oder 2, worin der Texaphyrin-Metallkomplex ein Yttrium-Texaphyrinkomplex ist.

5. Verfahren nach Anspruch 1 oder 2, worin das Licht eine Wellenlänge im Bereich von etwa 700 - 800 nm hat.

6. Verfahren nach Anspruch 1 oder 2, worin die Stufe der Belichtung des Metallkomplexes in Gegenwart von Sauerstoff durchgeführt wird.

7. Verfahren nach Anspruch 1, worin der Texaphyrin-Metallkomplex folgende Formel hat: 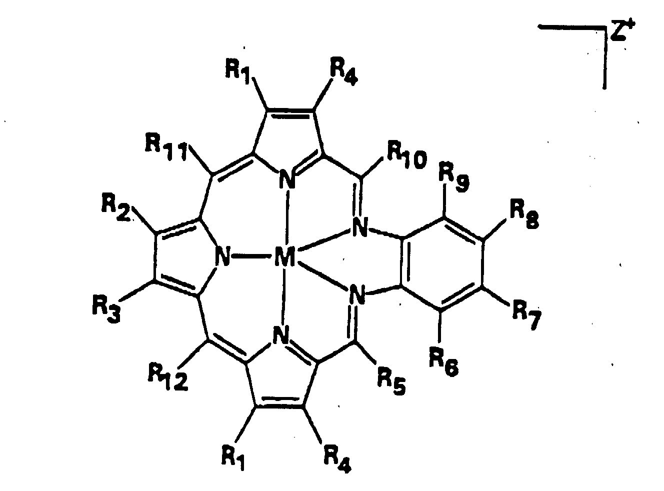 wobei
Mein zwei- oder dreiwertiges Metallkation ist mit kata-lytischer Wirkung für die Hydrolyse und Photospaltung der Ribonucleinsäure:
R₁ - R₄, R₇ und R₈ unabhängig voneinander Wasserstoff, Halogenid, Hydroxyl, Alkyl, Alkenyl, Alkinyl, Aryl, Halogenalkyl, Nitro, Formyl, Acyl, Hydroxyalkyl, Oxyalkyl, Oxyhydroxyalkyl, Hydroxyalkenyl, Hydroxyalkinyl, Saccharid, Carboxy, Carboxyalkyl, Carboxyamid, Carboxyamidalkyl, Amino, Aminoalkyl, ein sequenzdirigierende(s) bzw. (n) (site-directing) Molekül, eine katalytische Gruppe oder ein Verbindungsglied zu einem sequenzdirigierende(s) bzw. (n) (site-directing) Molekül oder einer katalytischen Gruppe sind;
R₆ und R₉ unabhängig voneinander ausgewählt werden aus den Gruppen von R₁ - R₄, R₇ und R₈ unter der Bedingung, daß das Halogenid nicht Iodid und das Halogenalkyl nicht Iodalkyl ist;
R₅ und R₁₀ - R₁₂ unabhängig voneinander Wasserstoff, Alkyl, Alkenyl, Alkinyl, Aryl, Hydroxyalkyl, Oxyalkyl, Oxyhydroxyalkyl, Hydroxyalkenyl, Hydroxyalkinyl, Carboxyalkyl, Carboxyamid, Carboxyamidalkyl, Amino, Aminoalkyl oder ein Verbindungsglied zu einem Saccharid, einem sequenzdirigierenden (site-directing) Molekül oder einer katalytischen Gruppe sind, und
Z ein ganzzahliger Wert unter oder gleich 5 ist.

8. verfahren nach Anspruch 2, worin der Texaphyrin-Metallkomplex folgende Formel hat: 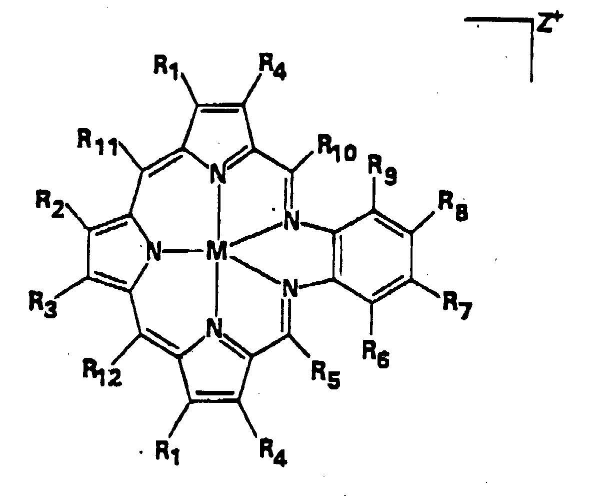 wobei
M ein zwei- oder dreiwertiges Metallkation ist mit katalytischer Wirkung für die Hydrolyse von Ribonucleinsäure und die Photospaltung von Desoxyribonucleinsäure;
R₁ - R₄, R₇ und R₈ unabhängig voneinander Wasserstoff, Halogenid, Hydroxyl, Alkyl, Alkenyl, Alkinyl, Aryl, Halogenalkyl, Nitro, Formyl, Acyl, Hydroxyalkyl, Oxyalkyl, Oxyhydroxyalkyl, Hydroxyalkenyl, Hydroxyalkinyl, Saccharid, Carboxy, Carboxyalkyl, Carboxyamid, Carboxyamidalkyl, Amino, Aminoalkyl, ein sequenzdirigierende(s) bzw. (n) (site-directing) Molekül, eine katalytische Gruppe oder ein Verbindungsglied zu einem sequenzdirigierenden (site-directing) Molekül oder einer katalytischen Gruppe sind;
R₆ und R₉ unabhängig voneinander ausgewählt werden aus den Gruppen von R₁ - R₄, R₇ und R₈ unter der Bedingung, daß das Halogenid nicht Iodid und das Halogenalkyl nicht Iodalkyl ist;
R₅ und R₁₀ -R₁₂ unabhängig voneinander Wasserstoff, Alkyl, Alkenyl, Alkinyl, Aryl, Hydroxyalkyl, Oxyalkyl, Oxyhydroxyalkyl, Hydroxyalkenyl, Hydxoxyalkinyl, Carboxyalkyl, Carboxyamid, Caxboxyamidalkyl, Amino, Aminoalkyl oder ein Verbindungsglied zu einem Saccharid, einem sequenzdirigierenden (site-directing) Molekül oder einer katalytischen Gruppe sind, und
Z ein ganzzahliger Wert unter oder gleich 5 ist.

9. Verfahren nach Anspruch 7 oder 8, worin M Yttrium(III) ist.

10. Verfahren nach Anspruch 9, worin R₁ (CH₂)₂CH₂OH ist; R₂ und R₃ CH₂CH₃ sind; R₄ CH₃ ist; R₅, R₆, R₈, R₉, R₁₀, R₁₁ und R₁₂ H sind; und R₇ OCH₂CONH-Oligonucleotid ist.

11. Verfahren nach Anspruch 7 oder 8, worin mindestens eines von R₁ - R₁₂ ein sequenzdirigierendes (site-directing) Molekül, eine katalytische Gruppe, ein Verbindungsglied zu einem sequenzdirigierenden (site-directing( Molekül oder ein Verbindungsglied zu einer katalytischen Gruppe ist.

12. Verfahren nach Anspruch 11, worin die katalytische Gruppe Imidazol, Guanidin, eine Aminosäure, ein Aminosäurederivat, eine Polyaminosäure, ein aminsubstituiertes Saccharid oder ein Texaphyrin-Metallkomplex ist.

13. Verfahren nach Anspruch 11, worin das sequenzdirigierte (site-directing) Molekül ein Oligonucleotid, ein Hormon, ein Antikörper, ein Peptid mit Affinität zu einem biologischen Rezeptor oder ein Saphirin-Molekül ist.

14. Verfahren nach Anspruch 7, worin mindestens eines von R₁ - R₁₂ ein sequenzdirigiertes (site-directing) Molekül ist, und das sequenzdirigierte (site-directing) Molekül ein Oligonucleotid ist, das eine Bindungsaffinität zur Ribonucleinsäure aufweist.

15. Verfahren nach Anspruch 13, worin das Oligonucleotid ein derivatisiertes Oligonucleotid oder ein Oligonucleotidanaloges ist.

16. Verfahren nach Anspruch 15, worin das derivatisierte Oligonucleotid aus der Gruppe ausgewählt wurde, die aus Methyl-phosphonaten, Phosphotriestern, Phosphorothioaten und Phosphoroamidaten besteht.

17. Verfahren nach Anspruch 15, worin das derivatisierte Oligonucleotid 2'-O-Alkyl-Oligoribonucleotid ist.

18. Verfahren nach Anspruch 13, worin das Oligonucleotid ein Antisense-Oligonucleotid ist.

19. Verwendung eines Texaphyrin-Metallkomplexes mit katalytischer Wirkung für die Hydrolyse und Photospaltung von Ribo-nucleinsäure bei der Herstellung einer pharmazeutischen Zusammensetzung zur Verwendung bei der Hydrolyse und Photospaltung von Ribonucleinsäure.

20. Verwendung nach Anspruch 19, worin der Texaphyrin-Metallkomplex folgende Formel hat: 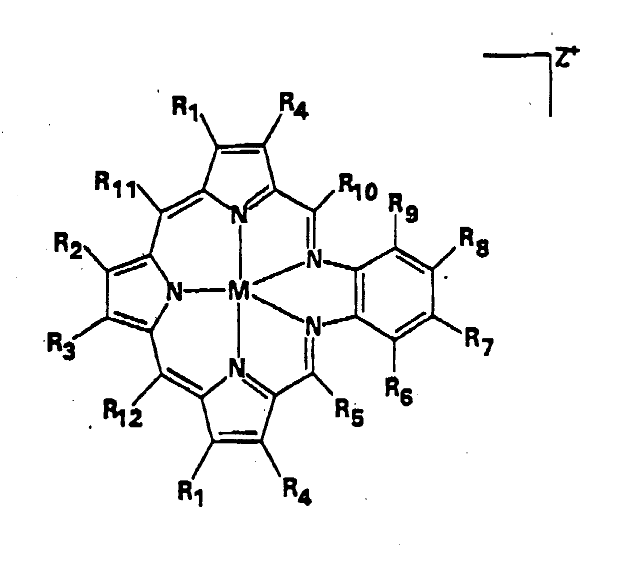 wobei
M ein zwei- oder dreiwertiges Metallkation ist mit katalytischer Wirkung für die Hydrolyse und Photospaltung der Ribonucleinsäure;
R₁ - R₄, R₇ und R₈ unabhängig voneinander Wasserstoff, Halogenid, Hydroxyl, Alkyl, Alkenyl, Alkinyl, Aryl, Halogenalkyl, Nitro, Formyl, Acyl, Hydroxyalkyl, Oxyalkyl, Oxyhydroxyalkyl, Hydroxyalkenyl, Hydroxyalkinyl, Saccharid, Carboxy, Carboxyalkyl, Carboxyamid, Carboxyamidalkyl, Amino, Aminoalkyl, ein sequenzdirigiertes (site-directing) Molekül, eine katalytische Gruppe oder ein Verbindungsglied zu einem sequenzdirigierten (site-directing) Molekül oder einer katalytischen Gruppe sind;
R₆ und R₉ unabhängig voneinander ausgewählt werden aus den Gruppen von R₁ - R₄, R₇ und R₈ unter der Bedingung, daß das Halogenid nicht Iodid und das Halogenalkyl nicht Iodalkyl ist;
R₅ und R₁₀ - R₁₂ unabhängig voneinander Wasserstoff, Alkyl, Alkenyl, Alkinyl, Aryl, Hydroxyalkyl, Oxyalkyl, Oxyhydroxyalkyl, Hydroxyalkenyl, Hydroxyalkinyl, Carboxyalkyl, Carboxyamid, Carboxyamidalkyl, Amino, Aminoalkyl oder ein Verbindungsglied zu einem Saccharid, einem sequenzdirigierten (site-directing) Molekül oder einer katalytischen Gruppe sind, und
Z ein ganzzahliger Wert unter oder gleich 5 ist.

21. Verwendung eines Texaphyrin-Metallkomplexes mit katalytischer Wirkung für die Hydrolyse von Ribonucleinsäure und die Photospaltung von Desoxyribonucleinsäure bei der Her-stellung einer pharmazeutischen Zusammensetzung zur Verwendung bei der Hydrolyse von Ribonucleinsäure und der Photospaltung von Desoxyribonucleinsäure.

22. Verwendung nach Anspruch 21, worin der Texaphyrin-Metallkomplex folgende Formel hat: 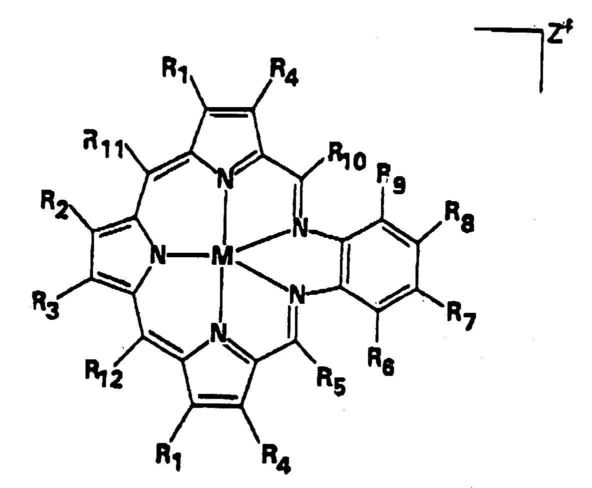 wobei
M ein zwei- oder dreiwertiges Metallkation ist mit katalytischer Wirkung für die Hydrolyse von Ribonucleinsäure und die Photospaltung von Desoxyribonucleinsäure;
R₁ - R₄, R₇ und R₈ unabhängig voneinander Wasserstoff, Halogenid, Hydroxyl, Alkyl, Alkenyl, Alkinyl, Aryl, Halogenalkyl, Nitro, Formyl, Acyl, Hydroxyalkyl, Oxyalkyl, Oxyhydroxyalkyl, Hydroxyalkenyl, Hydroxyalkinyl, Saccharid, Carboxy, Carboxyalkyl, Carboxyamid, Carboxyamidalkyl, Amino, Aminoalkyl, ein sequenzdirigiertes (site-directing) Molekül, eine katalytische Gruppe oder ein Verbindungsglied zu einem sequenzdirigierten (site-directing) Molekül oder einer katalytischen Gruppe sind;
R₆ und R₉ unabhängig voneinander ausgewählt werden aus den Gruppen von R₁ - R₄, R₇ und R₈ unter der Bedingung, daß das Halogenid nicht Iodid und das Halogenalkyl nicht Iodalkyl ist;
R₅ und R₁₀ - R₁₂ unabhängig voneinander Wasserstoff, Alkyl, Alkenyl, Alkinyl, Aryl, Hydroxyalkyl, Oxyalkyl, Oxyhydroxyalkyl, Hydroxyalkenyl, Hydroxyalkinyl, Carboxyalkyl, Carboxyamid, Carboxyamidalkyl, Amino, Aminoalkyl oder ein Verbindungsglied zu einem Saccharid, einem sequenzdirigierten (site-directing) Molekül oder einer katalytischen Gruppe sind, und
Z ein ganzzahliger Wert unter oder gleich 5 ist.

23. Verwendung nach Anspruch 20 oder 22, worin M Yttrium ist.

24. Verwendung nach Anspruch 23, worin R₁ (CH₂)₂CH₂OH ist, R₂ und R₃ CH₂CH₃ sind; R₄ CH₃ ist; R₅, R₆, R₈, R₉, R₁₀, R₁₁ und R₁₂ H sind und R₇ OCH₂CONH-Oligonucleotid ist.

## Revendications

1. Procédé permettant à la fois d'hydrolyser et de photocliver un acide ribonucléique, lequel procédé comporte :
- le fait de mettre l'acide ribonucléique en contact avec un complexe métallique de texaphyrine qui possède une activité catalytique dans les deux réactions d'hydrolyse et de photoclivage d'un acide ribonucléique ;
- le fait de faire incuber l'acide ribonucléique et le complexe métallique pendant suffisamment longtemps et dans des conditions appropriées pour hydrolyser des liaisons phosphodiester de l'acide ribonucléique ;
- et le fait d'exposer le complexe métallique de texaphyrine à de la lumière pendant suffisamment longtemps pour photocliver l'acide ribonucléique.

2. Procédé permettant à la fois d'hydrolyser un acide ribonucléique et de photocliver un acide désoxyribonucléique, lequel procédé comporte :
- le fait de mettre un mélange d'acide ribonucléique et d'acide désoxyribonucléique en contact avec un complexe métallique de texaphyrine qui possède une activité catalytique dans les deux réactions d'hydrolyse d'un acide ribonucléique et de photoclivage d'un acide désoxyribonucléique ;
- le fait de faire incuber le mélange et le complexe métallique pendant suffisamment longtemps et dans des conditions appropriées pour hydrolyser des liaisons phosphodiester de l'acide ribonucléique ;
- et le fait d'exposer le complexe métallique de texaphyrine à de la lumière pendant suffisamment longtemps pour photocliver l'acide désoxyribonucléique.

3. Procédé conforme à la revendication 1 ou 2, dans lequel le complexe métallique de texaphyrine est un complexe de texaphyrine et d'un métal diamagnétique.

4. Procédé conforme à la revendication 1 ou 2, dans lequel le complexe métallique de texaphyrine est un complexe de texaphyrine et d'yttrium.

5. Procédé conforme à la revendication 1 ou 2, dans lequel le domaine de longueurs d'onde de la lumière va à peu près de 700 à 800 nm.

6. Procédé conforme à la revendication 1 ou 2, dans lequel l'étape d'exposition du complexe métallique de texaphyrine est réalisée en présence d'oxygène.

7. Procédé conforme à la revendication 1, dans lequel le complexe métallique de texaphyrine a pour formule : 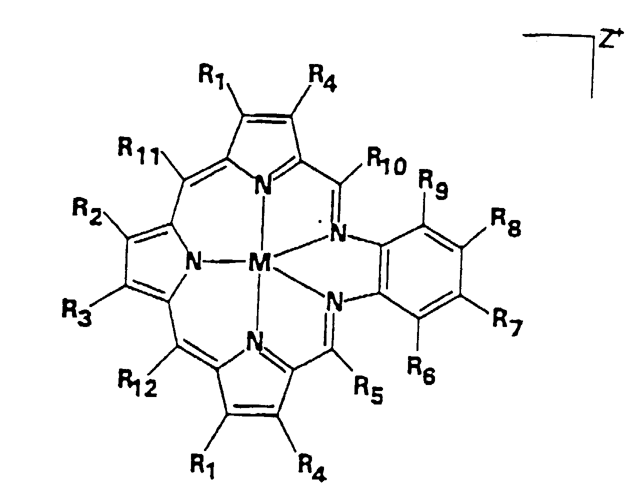 dans laquelle
M représente un cation d'un métal divalent ou un cation d'un métal trivalent, qui possède une activité catalytique dans les deux réactions d'hydrolyse et de photoclivage d'un acide ribonucléique ;
les symboles R₁ à R₄, R₇ et R₈ représentent chacun, indépendamment, un atome d'hydrogène ou d'halogène, un groupe hydroxy, alkyle, alcényle, alcynyle, aryle, halogénoalkyle, nitro, formyle, acyle, hydroxyalkyle, oxyalkyle, oxyhydroxyalkyle, hydroxyalcényle, hydroxyalcynyle, carboxy, carboxyalkyle, carboxamido, carboxamidoalkyle, amino ou aminoalkyle, le reste d'un saccharide ou d'une molécule de ciblage, un groupe catalytique, ou un chaînon de couplage à une molécule de ciblage ou à un groupe catalytique ;
R₆ et R₉ sont indépendamment choisis parmi les substituants indiqués à propos de R₁ à R₄, R₇ et R₈, sous réserve que l'atome d'halogène ne soit pas un atome d'iode et que le groupe halogénoalkyle ne soit pas un groupe iodoalkyle ;
les symboles R₅ et R₁₀ à R₁₂ représentent chacun, indépendamment, un atome d'hydrogène, un groupe alkyle, alcényle, alcynyle, aryle, hydroxyalkyle, oxyalkyle, oxyhydroxyalkyle, hydroxyalcényle, hydroxyalcynyle, carboxyalkyle, carboxamido, carboxamidoalkyle, amino ou aminoalkyle, ou un chaînon de couplage à un saccharide, à une molécule de ciblage ou à un groupe catalytique ; et
Z représente un nombre entier inférieur ou égal à 5.

8. Procédé conforme à la revendication 2, dans lequel le complexe métallique de texaphyrine a pour formule : 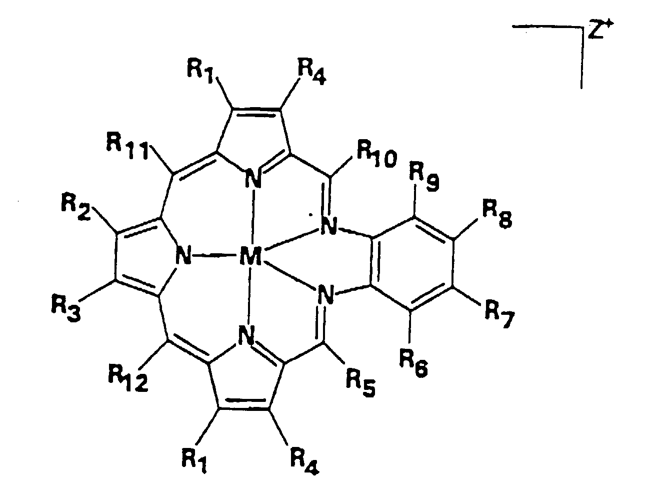 dans laquelle
M représente un cation d'un métal divalent ou un cation d'un métal trivalent, qui possède une activité catalytique dans les deux réactions d'hydrolyse d'un acide ribonucléique et de photoclivage d'un acide désoxyribonucléique ;
les symboles R₁ à R₄, R₇ et R₈ représentent chacun, indépendamment, un atome d'hydrogène ou d'halogène, un groupe hydroxy, alkyle, alcényle, alcynyle, aryle, halogénoalkyle, nitro, formyle, acyle, hydroxyalkyle, oxyalkyle, oxyhydroxyalkyle, hydroxyalcényle, hydroxyalcynyle, carboxy, carboxyalkyle, carboxamido, carboxamidoalkyle, amino ou aminoalkyle, le reste d'un saccharide ou d'une molécule de ciblage, un groupe catalytique, ou un chaînon de couplage à une molécule de ciblage ou à un groupe catalytique ;
R₆ et R₉ sont indépendamment choisis parmi les substituants indiqués à propos de R₁ à R₄, R₇ et R₈, sous réserve que l'atome d'halogène ne soit pas un atome d'iode et que le groupe halogénoalkyle ne soit pas un groupe iodoalkyle ;
les symboles R₅ et R₁₀ à R₁₂ représentent chacun, indépendamment, un atome d'hydrogène, un groupe alkyle, alcényle, alcynyle, aryle, hydroxyalkyle, oxyalkyle, oxyhydroxyalkyle, hydroxyalcényle, hydroxyalcynyle, carboxyalkyle, carboxamido, carboxamidoalkyle, amino ou aminoalkyle, ou un chaînon de couplage à un saccharide, à une molécule de ciblage ou à un groupe catalytique ; et
Z représente un nombre entier inférieur ou égal à 5.

9. Procédé conforme à la revendication 7 ou 8, dans lequel M représente un atome d'yttrium-(III).

10. Procédé conforme à la revendication 9, dans lequel R₁ représente un groupe -(CH₂)₂CH₂OH, R₂ et R₃ représentent des groupes -CH₂CH₃, R₄ représente un groupe -CH₃, R₅, R₆, R₈, R₉, R₁₀, R₁₁ et R₁₂ représentent des atomes d'hydrogène, et R₇ représente un reste -OCH₂CONH-oligonucléotide.

11. Procédé conforme à la revendication 7 ou 8, dans lequel au moins l'un des symboles R₁ à R₁₂ représente le reste d'une molécule de ciblage, un groupe catalytique, ou un chaînon de couplage à une molécule de ciblage ou à un groupe catalytique.

12. Procédé conforme à la revendication 11, dans lequel le groupe catalytique est un reste d'imidazole, de guanidine, d'acide aminé, de dérivé d'acide aminé, de poly(acide aminé), de saccharide à substituant amino, ou de complexe métallique de texaphyrine.

13. Procédé conforme à la revendication 11, dans lequel la molécule de ciblage est un oligonucléotide, une hormone, un anticorps, un peptide possédant une certaine affinité pour un récepteur biologique, ou une molécule de sapphyrine.

14. Procédé conforme à la revendication 7, dans lequel au moins l'un des symboles R₁ à R₁₂ représente le reste d'une molécule de ciblage, et cette molécule de ciblage est un oligonucléotide qui possède une certaine affinité pour ledit acide ribonucléique.

15. Procédé conforme à la revendication 13, dans lequel l'oligonucléotide est un dérivé d'oligonucléotide ou un analogue d'oligonucléotide.

16. Procédé conforme à la revendication 15, dans lequel le dérivé d'oligonucléotide est choisi dans l'ensemble que constituent les méthylphosphonates, les phosphotriesters, les phosphorothioates et les phosphoramidates.

17. Procédé conforme à la revendication 15, dans lequel le dérivé d'oligonucléotide est un 2'-O-alkyl-oligoribonucléotide.

18. Procédé conforme à la revendication 13, dans lequel l'oligonucléotide est un oligonucléotide anti-sens.

19. Emploi d'un complexe métallique de texaphyrine qui possède une activité catalytique dans les deux réactions d'hydrolyse et de photoclivage d'un acide ribonucléique, dans la préparation d'une composition pharmaceutique destinée à être utilisée pour à la fois hydrolyser et photocliver un acide ribonucléique.

20. Emploi, conforme à la revendication 19, d'un complexe métallique de texaphyrine de formule : 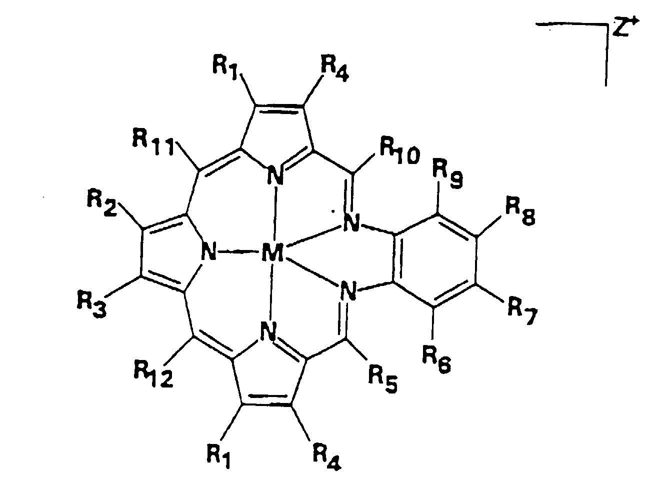 dans laquelle
M représente un cation d'un métal divalent ou un cation d'un métal trivalent, qui possède une activité catalytique dans les deux réactions d'hydrolyse et de photoclivage d'un acide ribonucléique ;
les symboles R₁ à R₄, R₇ et R₈ représentent chacun, indépendamment, un atome d'hydrogène ou d'halogène, un groupe hydroxy, alkyle, alcényle, alcynyle, aryle, halogénoalkyle, nitro, formyle, acyle, hydroxyalkyle, oxyalkyle, oxyhydroxyalkyle, hydroxyalcényle, hydroxyalcynyle, carboxy, carboxyalkyle, carboxamido, carboxamidoalkyle, amino ou aminoalkyle, le reste d'un saccharide ou d'une molécule de ciblage, un groupe catalytique, ou un chaînon de couplage à une molécule de ciblage ou à un groupe catalytique ;
R₆ et R₉ sont indépendamment choisis parmi les substituants indiqués à propos de R₁ à R₄, R₇ et R₈, sous réserve que l'atome d'halogène ne soit pas un atome d'iode et que le groupe halogénoalkyle ne soit pas un groupe iodoalkyle ;
les symboles R₅ et R₁₀ à R₁₂ représentent chacun, indépendamment, un atome d'hydrogène, un groupe alkyle, alcényle, alcynyle, aryle, hydroxyalkyle, oxyalkyle, oxyhydroxyalkyle, hydroxyalcényle, hydroxyalcynyle, carboxyalkyle, carboxamido, carboxamidoalkyle, amino ou aminoalkyle, ou un chaînon de couplage à un saccharide, à une molécule de ciblage ou à un groupe catalytique ; et
Z représente un nombre entier inférieur ou égal à 5.

21. Emploi d'un complexe métallique de texaphyrine qui possède une activité catalytique dans les deux réactions d'hydrolyse d'un acide ribonucléique et de photoclivage d'un acide désoxyribonucléique, dans la préparation d'une composition pharmaceutique destinée à être utilisée pour à la fois hydrolyser un acide ribonucléique et photocliver un acide désoxyribonucléique.

22. Emploi, conforme à la revendication 21, d'un complexe métallique de texaphyrine de formule : 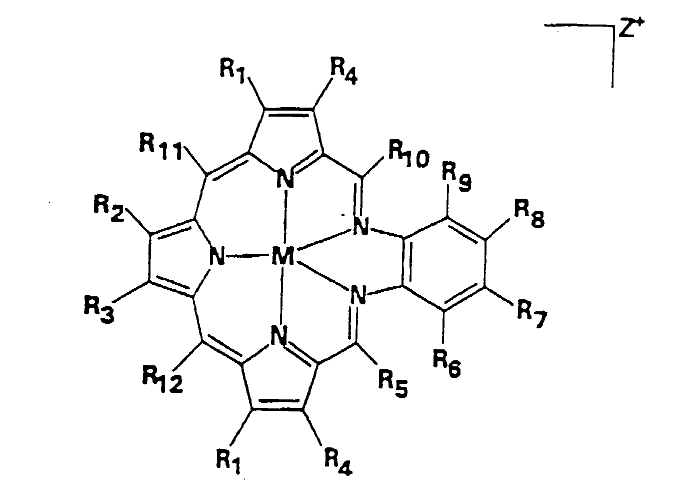 dans laquelle
M représente un cation d'un métal divalent ou un cation d'un métal trivalent, qui possède une activité catalytique dans les deux réactions d'hydrolyse d'un acide ribonucléique et de photoclivage d'un acide désoxyribonucléique ;
les symboles R₁ à R₄, R₇ et R₈ représentent chacun, indépendamment, un atome d'hydrogène ou d'halogène, un groupe hydroxy, alkyle, alcényle, alcynyle, aryle, halogénoalkyle, nitro, formyle, acyle, hydroxyalkyle, oxyalkyle, oxyhydroxyalkyle, hydroxyalcényle, hydroxyalcynyle, carboxy, carboxyalkyle, carboxamido, carboxamidoalkyle, amino ou aminoalkyle, le reste d'un saccharide ou d'une molécule de ciblage, un groupe catalytique, ou un chaînon de couplage à une molécule de ciblage ou à un groupe catalytique ;
R₆ et R₉ sont indépendamment choisis parmi les substituants indiqués à propos de R₁ à R₄, R₇ et R₈, sous réserve que l'atome d'halogène ne soit pas un atome d'iode et que le groupe halogénoalkyle ne soit pas un groupe iodoalkyle ;
les symboles R₅ et R₁₀ à R₁₂ représentent chacun, indépendamment, un atome d'hydrogène, un groupe alkyle, alcényle, alcynyle, aryle, hydroxyalkyle, oxyalkyle, oxyhydroxyalkyle, hydroxyalcényle, hydroxyalcynyle, carboxyalkyle, carboxamido, carboxamidoalkyle, amino ou aminoalkyle, ou un chaînon de couplage à un saccharide, à une molécule de ciblage ou à un groupe catalytique ; et
Z représente un nombre entier inférieur ou égal à 5.

23. Emploi conforme à la revendication 21 ou 22, dans lequel M représente un atome d'yttrium.

24. Emploi conforme à la revendication 23, dans lequel R₁ représente un groupe -(CH₂)₂CH₂OH, R₂ et R₃ représentent des groupes -CH₂CH₃, R₄ représente un groupe -CH₃, R₅, R₆, R₈, R₉, R₁₀, R₁₁ et R₁₂ représentent des atomes d'hydrogène, et R₇ représente un reste -OCH₂CONH-oligonucléotide.


### SEQUENCE LISTING

### (1) GENERAL INFORMATION:

(i) APPLICANT: Pharmacyclics, Inc.
   Board of Regents, The University of Texas System
(ii) TITLE OF INVENTION: MULTI-MECHANISTIC CHEMICAL CLEAVAGE
   USING CERTAIN METAL COMPLEXES
(iii) NUMBER SEQUENCES: 6
(iv) CORRESPONDENCE ADDRESS:
   (A) ADDRESSEE: Akin, Gump, Strauss, Hauer & Feld, L.L.P.
   (B) STREET: 816 Congress Avenue, Suite 1900
   (C) CITY: Austin
   (D) STATE: Texas
   (E) COUNTRY: United States of America
   (F) ZIP: 78701
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.30
(vi) CURRENT APPLICATION DATA:
   (A) APPLICATION NUMBER: PCT unknown
   (B) FILING DATE: Concurrently herewith
   (C) CLASSIFICATION: Unknown
(viii) ATTORNEY/AGENT INFORMATION:
   (A) NAME: Norberg, Gloria L.
   (B) REGISTRATION NUMBER: 36,706
   (C) REFERENCE/DOCKET NUMBER: 43414.0007
(ix) TELECOMMUNICATION INFORMATION:
   (A) TELEPHONE: ((512) 499-6200
   (B) TELEFAX: (512) 499-6290
   (C) TELEX: 79-0924

### (2) INFORMATION FOR SEQ ID NO:1:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:1:
   CATCTGTGAG CCGGGTGTTG 20

### (2) INFORMATION FOR SEQ ID NO:2:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:2:
   CTCGGCCATA GCGAATGTTC 20

### (2) INFORMATION FOR SEQ ID NO:3:

(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:3:
   CAACACCCGG CTCACAGATG AAGTCTCCAA AATAAA 36

### (2) INFORMATION FOR SEQ ID NO:4:

(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:4:
   CAACACCCGG CUCACAGAUG AAGUCUCCAA AAUAAA 36

### (2) INFORMATION FOR SEQ ID NO:5:

(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:5:
   ACAGAACATT CGCTATGGCC GAGTGGAGAG ACCGCG 36

### (2) INFORMATION FOR SEQ ID NO:6:

(i) SEQUENCE CHARACTERISTICS:
   (A) LENGTH: 36 base pairs
   (B) TYPE: nucleic acid
   (C) STRANDEDNESS: single
   (D) TOPOLOGY: linear
(ii) MOLECULE TYPE: other nucleic acid
   (A) DESCRIPTION: /desc = "DNA"
(xi) SEQUENCE DESCRIPTION: SEQ ID NO:6:
   ACAGAACAUU CGCUAUGGCC GAGUGGAGAG ACCGCG 36